# EUROPÄISCHE PATENTSCHRIFT

(11) **EP 2 215 244 B1**
(45) Veröffentlichungstag und Bekanntmachung des Hinweises auf die Patenterteilung: **01.11.2017**
(21) Anmeldenummer: 08844284.3
(22) Anmeldetag: 24.10.2008
(51) Int. Cl.: C12P 7/66

(54) **VERFAHREN ZUR STEIGERUNG DES CO-ENZYM Q10-GEHALTS VON PHOTOTROPHEN MIKROORGANISMEN**
METHOD FOR INCREASING THE CO-ENZYME Q10 CONTENT OF PHOTOTROPHIC MICROORGANISMS
PROCÉDÉ D'AUGMENTATION DE LA TENEUR EN CO-ENZYME Q10 DE MICRO-ORGANISMES PHOTOTROPES

(30) Priorität: 30.10.2007 CH 1682007
(43) Veröffentlichungstag der Anmeldung: 11.08.2010
(73) Patentinhaber: Sanbo GmbH, 6340 Baar (CH)
(72) Erfinder: DÜRR, Oliver, CH-6340 Baar (CH); BUCHHOLZ, Rainer, 91093 Hessdorf (DE); LANGE, Harald, 13403 Berlin (DE); KLEIN, Barbara, Christina, Angela, Petra, 91058 Erlangen (DE); WALTER, Christian, 91056 Erlangen (DE)
(74) Vertreter: Schmauder & Partner AG Patent- & Markenanwälte VSP
(86) Internationale Anmeldenummer: PCT/CH2008/000450
(87) Internationale Veröffentlichungsnummer: WO 2009/055951

(56) Entgegenhaltungen:
- YOSHIDA HAJIME ET AL: "Production of ubiquinone-10 using bacteria" JOURNAL OF GENERAL AND APPLIED MICROBIOLOGY, Bd. 44, Nr. 1, Februar 1998 (1998-02), Seiten 19-26, XP009003546 ISSN: 0022-1260
- JIN-HO CHOI ET AL: "Biotechnological production and applications of coenzyme Q10" APPLIED MICROBIOLOGY AND BIOTECHNOLOGY, SPRINGER, BERLIN, DE, Bd. 68, Nr. 1, 1. Juli 2005 (2005-07-01), Seiten 9-15, XP019331893 ISSN: 1432-0614
- WALTER C ET AL: "Monoseptic cultivation of phototrophic microorganisms-development and scale-up of a photobioreactor system with thermal sterilization" BIOMOLECULAR ENGINEERING, ELSEVIER, NEW YORK, NY, US, Bd. 20, Nr. 4-6, 1. Juli 2003 (2003-07-01), Seiten 261-271, XP004446922 ISSN: 1389-0344 in der Anmeldung erwähnt
- LEE YUAN-KUN: "Microalgal mass culture systems and methods: Their limitation and potential" JOURNAL OF APPLIED PHYCOLOGY, Bd. 13, Nr. 4, August 2001 (2001-08), Seiten 307-315, XP002514617 ISSN: 0921-8971 in der Anmeldung erwähnt
- CHOI G S ET AL: "Restricted electron flux increases coenzyme Q10 production in Agrobacterium tumefaciens ATCC4452", PROCESS BIOCHEMISTRY, ELSEVIER, NL, vol. 40, no. 10, 1 October 2005 (2005-10-01), pages 3225-3229, XP025306621, ISSN: 1359-5113, DOI: 10.1016/J.PROCBIO.2005.03.038 [retrieved on 2005-10-01]

## Beschreibung

Die Erfindung betrifft ein Verfahren mit den oberbegrifflichen Merkmalen des Anspruchs 1.

### Ubiquinon (Q₁₀)

Ubiquinon (2,3-Dimethoxy-5-Methyl-6-Multiprenyl-1,4-Benzoquinon, Co-Enzym Q₁₀ bzw. Q10) ist aufgrund seiner antioxidativen Eigenschaften von besonderer Bedeutung für nahezu alle lebenden Zellen. Ubiquinon tritt in den meisten aeroben Mikroorganismen und in allen Tieren auf (Langsjoen, 1988 ; Ishii et *al.,* 2004). Auch für den menschlichen Organismus ist Q₁₀ essentiell (Langsjoen et *al.,* 1988).

Bei dem Co-Enzym Q₁₀ handelt es sich um ein fettlösliches Provitamin, das eine Strukturverwandtschaft zu Vitamin K aufweist (Shekelle and Morton, 2003). Ubiquinon stellt einen wichtigen Bestandteil der mitochondrialen Atmungskette dar. Es fungiert als Elektronentransportsystem und dient somit der ATP-Generierung. Dabei werden Elektronen ausgehend von NADH CoQ₁₀ -Reduktase und Succinate CoQ₁₀ -Reduktase auf QH₂-Cytochrome C Oxireduktase übertragen (Ishii *et al.,* 2004). Für diesen Sachverhalt ist die Quinongruppe des Moleküls verantwortlich, wohingegen die Isoprenseitenkette den hyrophoben Charakter definiert. Daher ist Ubiquinon ausschliesslich in den lipidreichen Regionen der Zelle zu finden (Lass *et al.,* 1997). Obwohl die innere Mitochondrienmembran die höchste Konzentration an Ubiquinon aufweist, ist Q₁₀ auch in anderen Zellmembranen weit verbreitet und übt mehrere Aufgaben in der Zelle aus. Neben seiner Funktion im Elektronentransportsystem und in der ATP-Produktion, stellt Ubiquinon das einzig bekannte endogen synthetisierte, fettlösliche Antioxidans dar (Shekelle and Morton, 2003; Cenedella *et al.,* 2005). Es dient in der Zelle als Radikalfänger und schützt daher mehrfach ungesättigte Fettsäuren, Membranproteine und DNA vor oxidativer Schädigung (Cenedella *et al.,* 2005; Chuang *et al.,* 2003). Als Antioxidans dient es der Neutralisierung schädlicher freier Radikale, die während der Energieproduktion in der Zelle entstehen (West, 2001).

Ubiquinon fällt somit die essentielle Rolle der Neutralisierung der Reactive Oxygen

Species (ROS) zu. Diese werden in den Mitochondrien als Nebenprodukt der ATP-Produktion gebildet und würden in Abwesenheit von Q₁₀ durch oxidative Schädigung der Zellstrukturen eine erhebliche Gefahr für die Mitochondrien darstellen. Ubiquinon ist in der Lage, Lipid-Peroxidation zu inhibieren, wenn Zellmembrane und Low-Density Lipoproteine *ex vivo* oxidierenden Bedingungen ausgesetzt sind; dabei wird Q₁₀ als vorrangiges Antioxidans genutzt (Higdon and Pauling, 2003). Es wird zudem vermutet, dass Q₁₀ einem Absterben von Dopaminproduzierenden Zellen entgegenwirkt.

Ubiquinon (Q₁₀) hat in den Zellen auch die Aufgabe der Wiederherstellung von oxidiertem Alpha-Tocopherol (Cenedella et *al.,* 2005). Alpha-Tocopherol stellt dabei neben Ubiquinon das wichtigste fettlösliche Antioxidans in Membranen und Lipoproteinen dar (Higdon and Pauling, 2003). Die Konzentration von Ubiquinon im Körper eines erwachsenen Menschen beträgt, abhängig vom Körpergewicht, ca. 2 Gramm. Q₁₀ ist aufgrund des hohen Energiebedarfs in erheblichen Konzentrationen in Herzmuskelzellen zu finden. Obwohl Ubiquinon in einer grossen Vielzahl von Lebensmitteln wie Rindfleisch, Sojaöl, Sardinen oder Erdnüssen vorhanden ist, wird der Körper quantitativ durch körpereigenes Co-Enzym Q₁₀ versorgt. Die Produktion von Q₁₀ im menschlichen Körper nimmt ab einem Alter von ca. 21 Jahren jedoch kontinuierlich ab. Mit etwa 40 Jahren beträgt die Q₁₀-Konzentration im Herz nur noch ungefähr 80 % und mit 80 Jahren nur noch annähernd 60 % der eines 20-Jährigen (Langsjoen 1988; Shekelle and Morton, 2003; Vormann, 1999). Eine unausgewogene Ernährung und Stress führen zusätzlich zu einer Verminderung von Ubiquinon im Körper. Oxidativer Stress, verursacht durch UV-Strahlung oder freie Radikale, führt zu Zelloxidation und somit zu Alterungsprozessen und frühzeitiger Hautalterung. Dies hängt mit der Abnahme endogener Zell-Antioxidantien im Alter zusammen.

In Studien konnte nachgewiesen werden, dass auf die Haut aufgetragenes Ubiquinon in die Schichten der Epidermis eindringt und dort den Oxidationsgrad verringert. Untersuchungen konnten weiterhin einen positiven Einfluss von Co-Enzym Q₁₀ auf UV A-verursachten, oxidativen Stress in humanen Keratinozyten und bei der Prävention oxidativer DNA-Schädigung nachweisen. Der reduzierten Resistenz gegen UV-Strahlung kann somit durch Oberflächenapplikation von Q₁₀ entgegengewirkt werden. Zudem wurde belegt, dass durch Q₁₀-Applikation eine Verringerung von Hautfalten erzielt wird. Daraus resultiert ein hohes Potential für Ubiquinon in der Prävention frühzeitiger Hautalterung (Blatt *et al.,* 1999; Hoppe *et al.,* 1999).

### Mikroalgen

Mikroalgen sind phototrophe Mikroorganismen, die mit relativ hohen Ausbeuten an Biotrockenmasse von ca. 30g m⁻² d⁻¹ unter photoautotrophen (Licht plus CO₂), mixotrophen (Licht plus organische Kohlenstoffquelle) oder heterotrophen (ohne Licht) Bedingungen kultiviert werden können. Die Kultivierung unter photoautotrophen Bedingungen ist besonders kostengünstig, da lediglich Wasser, Mineralsalze und Licht benötigt werden. Bei mixotropher Kulturführung können die Wachstumsausbeuten gesteigert werden. Viele der ökonomisch wichtigen Mikroalgen sind in der Lage, Lichtenergie und organischen Kohlenstoff gleichzeitig zu verwenden. Zum Teil wird dabei die Energie aus dem Licht für den Glucose-Metabolismus genutzt. Bei manchen Spezies setzt sich die maximale spezifische Wachstumsrate für das heterotrophe und das photoautotrophe Wachstum zusammen. Im Vergleich zu den autotrophen Wachstumsbedingungen ergibt sich durch den Zusatz von organischen Kohlenstoffen ein besseres Wachstum (Lee, 2001). Zudem tritt bei phototropher Kultivierung häufig das Problem der gegenseitigen Abschirmung der Mikroalgenzellen auf. Somit verläuft der Lichteintrag pro Zelle entgegengesetzt proportional zur Zelldichte. Zudem kommt es zu einer exponentiellen Abschwächung des Lichts mit dem Weg (Rarbach, 1999), was sich negativ auf das Wachstum auswirkt. Eine Minderung dieses Effektes kann durch mixotrophe Prozessführung erzielt werden.

Zur Produktion von Co-Enzym Q₁₀ werden verschiedene Verfahren vorgeschlagen. So zeigt beispielsweise die WO2005/005650 ein Verfahren zur Gewinnung von Co-Enzym Q₁₀ aus Mikroorganismen der Art *Rhodobacter,* die mit dem Mevalonat-Operon aus *Paracoccus zeaxanthinifaciens* transformiert wurden. Park *et al.* (2005) berichten eine Produktion des Co-Enzyms Q₁₀ in einem rekombinanten *Escherichia coli*-Stamm, der das Decaprenyl-Diphosphat-Synthase-Gen aus *Gluconobacter suboxydans* exprimiert. Die WO2006/136311 zeigt ein System zur Expression von Genen aus dem Q₁₀-Biosyntheseweg in Hefen der Gattung *Sporidiobolus.* Ha *et al.* (2007) berichten über eine Erhöhung des Co-Enzym Q₁₀-Gehaltes in einer *Agrobacterium tumefaciens*-Mutante durch Anpassung der Saccharose-Konzentration im Kulturmedium. Die US 6,506,915 stellt ein Verfahren zur synthetischen Herstellung von Co-Enzym Q₁₀ mit Geraniol als Ausgangsmaterial vor.

Yoshida et al. ("Production of ubiquinone-10 using bacteria", Journal of General and Applied Microbiology, Bd. 44, Nr. 1, Februar 1998, 19-26) berichten über ein Verfahren zur Steigerung des Co-Enzym Q₁₀-Gehalts von *Rhodobacter spaeroides* durch Mutagenese und Verringerung der Luftzufuhr im Kulturgefäss.

Nachteilig an den oben beschriebenen Verfahren ist, dass diese zur Produktion von Co-Enzym Q₁₀ entweder genetisch modifizierte Organismen (GMO) verwenden oder versuchen, ein nicht-natürliches Co-Enzym Q₁₀ auf synthetischem Wege herzustellen.

Aufgabe der vorliegenden Erfindung ist es daher, ein Verfahren zur Verfügung zu schaffen, mit dem Co-Enzym Q₁₀ wirtschaftlich konkurrenzfähig, in gleichbleibend hoher Qualität und mit hoher Ausbeute an Naturstoff in genetisch nicht transformierten Organismen zur Verfügung gestellt werden kann.

Diese Aufgabe wird gelöst durch ein Verfahren gemäss Anspruch 1. Vorteilhafte Weiterbildungen der Erfindung sind Gegenstand der abhängigen Ansprüche.

Das erfindungsgemässe Verfahren dient zur Steigerung des Co-Enzym Q₁₀-Gehalts von phototrophen Mikroorganismen. Die phototrophen Mikroorganismen werden dabei in einem Kulturmedium in einem Bioreaktor oder Photobioreaktor unter Standardbedingungen kultiviert. Das Wachstum der Mikroorganismen weist dabei zu Beginn zunächst eine exponentielle Wachstumsphase mit Zunahme der Zellzahlen im Medium auf. Nach Erreichen eines artspezifischen und/oder durch die Kulturbedingungen bestimmten Grenzwertes geht das Wachstum in eine stationäre Wachstumsphase über. Um eine Steigerung des Co-Enzym Q₁₀-Gehalts in den phototrophen Mikroorganismen zu erreichen, ist das erfindungsgemässe Verfahren gekennzeichnet durch eine:
Induktion von oxidativem Stress durch Co-Inkubation der stressinduzierenden Substanzen 13S-Hydroperoxy-9Z,11E-octadecadiensäure und Eisen, insbesondere Fe2+, mit den Mikroorganismen im Kulturmedium und/oder Erhöhung der Oberflächenbestrahlungsstärke am Bioreaktor.

In einer Ausführungsform handelt es sich um Mitochondrien aufweisende Mikroorganismen und es erfolgt als weiterer Schritt eine
Zugabe von Oleat zum Kulturmedium.

Günstigerweise kann die Zugabe von Oleat zum Kulturmedium dabei entweder gleichzeitig mit der Induktion von oxidativem Stress oder zeitlich von dieser versetzt im späteren Verlauf der Kultivierung der Mikroorganismen erfolgen.

Mit geschätzten 50000 Spezies stellen phototrophe Mikroorganismen eine bedeutende Quelle für verschiedenste Naturstoffe dar. Mikroalgen produzieren beispielsweise mehrfach ungesättigte Fettsäuren (z.B. Omega-3-Fettsäuren wie DHA und EPA), Antioxidantien sowie biologisch aktive Substanzen mit antibiotischen, antiviralen oder antitumoralen Wirkungsweisen (Walter et *al.,* 2003). Als besonders günstig wird es daher im Zusammenhang mit dem erfindungsgemässen Verfahren angesehen, wenn als phototrophe Mikroorganismen Mikroalgen verwendet werden, die aus den Abteilungen Rhodophyta, Chlorophyta oder Haptophyta ausgewählt sind. Hierbei sind insbesondere aus *Porphyridium purpureum,* und *Cricosphaera carterae* hohe Ausbeuten an Co-Enzym Q₁₀ erzielbar. Darüber hinaus ist auch die Verwendung von Mikroalgen der Arten *Chlorella vulgaris* und *Pavlova lutheri* für die Gewinnung von des Co-Enzyms Q₁₀ möglich.

Das mixotrophe Wachstum der Mikroalgen erfolgt bei einer Kultivierung unter für die jeweilige Art ermittelten Bedingungen. So erfolgt beispielsweise standardmässig mixotrophes Wachstum der Mikroalge *Cricosphaera carterae* in Photobioreaktoren bei einer Oberflächenbestrahlungsstärke I₀ von 90-100 µE·m⁻²·s⁻¹. Der CO₂-Volumenanteil der zugeführten Luft liegt bei 1,0 % CO₂ und das Kulturmedium wird mit 70 rpm bei einer Temperatur von 20 °C gerührt. Als Kulturmedium dient ein Salzmedium (M5 + Vit. B1), das nach folgendem Protokoll hergestellt wird:

| Komponente | Stammlösung | Menge[L⁻¹] |
|---|---|---|
| KNO₃ | 5 g·500 mL⁻¹ | 20 mL |
| K₂HPO₄ | 0,5 g·500 mL⁻¹ | 20 mL |
| MgSO₄·7H₂O | 0,5 g·500 mL⁻¹ | 20 mL |
| Mikronährlösung III | | 5 mL |
| Tropic Marin (5-fach) | 166 g·1 L⁻¹ | 181 mL |
| Reinstwasser | - | 723 mL (M5a: 673 mL) |
| Autoklavieren, anschliessend werden nachfolgende Substanzen steril zugegeben | | |
| Erdextrakt | - | 30 mL |
| Vit. B₁₂ | 2,5 mg·500 mL | 1 mL |

Die im Protokoll angegebene Mikronährlösung III enthält dabei die folgenden Komponenten in den angegebenen Konzentrationen:

| KOMPONENTE | STAMMLÖSUNG | MENGE [L⁻¹] |
|---|---|---|
| ZNSO₄·7H₂O | 10,0 MG·10,0 ML⁻¹ | 1,00 ML |
| MNSO₄·4H₂O | 10,0 MG·10,0 ML⁻¹ | 2,00 ML |
| H₃BO₃ | 2,00 MG·10,0 ML⁻¹ | 5,00 ML |
| C(NO₃)₂·6H₂O | 2,00 MG·10,0 ML⁻¹ | 5,00 ML |
| NA₂MOO₄·2H₂O | 2,00 MG·10,0 ML⁻¹ | 5,00 ML |
| CUSO₄·5H₂O | 0,50 MG·100 ML⁻¹ | 1,00 ML |
| VE-H₂O | - | 881 ML |
| EDTA (TITRIPLEX III) | - | 0,40 G |
| FESO₄·7H₂O | - | 0,70 G |
| EDTA (TITRIPLEX III) | - | 0,40 G |

Dem Medium werden als organische Kohlenstoffquellen zusätzlich folgende Substanzen beigefügt:
▪ Glucose 3,00 g·L⁻¹ (1,7·10⁻² mol·L⁻¹)
▪ Glycerin 1,53 g·L⁻¹ (1,7·10⁻² mol·L⁻¹)
▪ Saccharose 5,70 g·L⁻¹ (1,7·10⁻² mol·L⁻¹)
▪ Fructose 3,00 g·L⁻¹ (1,7·10⁻² mol·L⁻¹)
▪ Fleischextrakt 3,00 g·L⁻¹
▪ sowie Natriumacetat 1,37 g·L⁻¹ (1,7·10⁻² mol·L⁻¹).

Für die Kultivierung von *P. purpureum* in Photobioreaktoren wiederum, wird standardmässig das ASW-Medium nach Jones *et al.,* (1963) verwendet, das nach folgendem Protokoll hergestellt wird:

| Komponente | Menge [L⁻¹] |
|---|---|
| NaCl | 27,0 g |
| MgSO₄·7H₂O | 6,60 g |
| MgCl₂·6H₂O | 5,60 g |
| CaCl₂·2H₂O | 1,50 g |
| KNO₃ | 1,00 g |
| KH₂PO₄ | 0,07 g |
| NaHCO₃ | 0,04 g |
| Tris-HCl-Puffer pH 7,6 | 20 mL |
| Spurenelementlösung für ASW | 1,00 mL |
| Eisen-Chelat-Lösung pH 7,6 | 1,00 mL |
| Reinstwasser | 987 mL |

Die im Protokoll angegebene Spurenelementlösung enthält dabei die folgenden Komponenten in den angegebenen Konzentrationen:

| Komponente | Menge [100 mL⁻¹] |
|---|---|
| ZnCl₂ | 4,00 mg |
| H₃BO₃ | 60,0 mg |
| CoCl₂·6H₂O | 1,50 mg |
| CuCl₂·2H₂O | 4,00 mg |
| MnCl₂·4H₂O | 40,0 mg |
| (NH₄)₆Mo₇O₂₄·4H₂O | 37,0 mg |
| Reinstwasser | 100 mL |

Die Temperatur des Mediums wird auf 25 °C eingestellt. Der Gasvolumenstrom beträgt 3,2 NL·min⁻¹ und ist mit 2,5 % CO₂ angereichert. Bei der Kultivierung von *P. purpureum* ist zusätzlich der zeitliche Verlauf der Prozessführung und die Prozessdauer zu beachten. So erfolgt die Kultivierung beispielsweise in den ersten 117h im Batch-Betrieb, d.h. in absatzweiser Kultivierung, worauf für weitere 465h zu einer kontinuierlichen Kultivierung übergegangen wird, um die Algen danach nochmals für 183h im Batch-Betrieb zu kultivieren. Die Kultivierung wird bei einer durchschnittlichen Oberflächenbestrahlungsstärke I₀ von 100 µE·m⁻²·s⁻¹ durchgeführt.

Als Photobioreaktoren eignen sich alle bekannten Reaktoren, die thermisch sterilisierbar sind und über entsprechende Steuerungsmöglichkeiten für die Kultivierungsparameter verfügen. Als besonders geeignet erweisen sich in diesem Zusammenhang die Photobioreaktoren vom Typ Medusa im 10 L- und 25 L-Massstab. Der 10 L-Reaktor besteht dabei im Gegensatz zum 25 L-Photobioreaktor aus zwei Glasrohren (Borosilikat 3.3, Fa. QVF), die mit einem U-Rohr verbunden sind. Der 25 L-Medusareaktor ist aus 4 Glasrohren aufgebaut, von denen je zwei mit einem U-Rohr verbunden sind. Die Glasrohre sind über den Kopfraum verbunden. Der Kopfraum des 10 L-Reaktors ist im Gegensatz zum 25 L-Reaktor, dessen Kopfraum aus Glas (Borosilikat 3.3, Fa. QVF) besteht, aus Edelstahl (VA 1.4301) gefertigt. Die Begasungseinrichtung des 10 L-Reaktors ist seitlich an einem Glasrohr, die des 25 L-Reaktors unten am U-Rohr angebaut. Die Anordnung ist dabei so gestaltet, dass die Prozessluft in je ein Rohr einer Schlaufe eingeleitet wird. Somit wird durch die entstehende hydrostatische Druckdifferenz eine Strömung erzeugt, die eine Durchmischung im Reaktor bewirkt. Am Reaktordeckel des 25 L-Reaktors bzw. im Kopfraum des 10 L-Reaktors sind Anschlüsse für Messsonden (pH und pO₂), Überdruckventil, Manometer, die Abluftstrecke und Stutzen zur Inokulation montiert. Die Temperierung der Photobioreaktoren erfolgt mit einem Kryostaten (TRONIC, Fa. Störk). Die Lichtversorgung erfolgt über Leuchtstofflampen (TLD 58 W/840, Fa. Philips - 10 L-Reaktor; Lumilux Plus Coolwhite, L 58 W/21-840, Fa. Osram - 25 L-Reaktor). Dabei entsteht für den 10 L-Reaktor als direkt bestrahltes Oberflächen-Volumen/Verhältnis (A/V) von 48 m⁻¹ und für den 25 L-Reaktorvon 38,5 m⁻¹ (Walter, 1999; König, 2006).

Hinsichtlich einer Steigerung der Bildung von Q₁₀ in den im Verfahren eingesetzten Mikroorganismen wirkt sich sowohl eine Erhöhung der Oberflächenbestrahlungsstärke als auch die Co-Inkubation von oxidativen Stress induzierenden Substanzen positiv auf die extrahierbare Qio-Menge in der gebildeten Biomasse aus. Dies ist zum auf eine durch die höheren Oberflächenbestrahlungsstärken erhöhte Photooxidation, zum anderen auf eine durch die co-inkubierten Substanzen erzeugte Lipidperoxidation, die den oxidativen Stress steigert, zurückzuführen.

Neben einer Induktion von oxidativem Stress umfasst das Verfahren bei Mitochondrien aufweisenden Mikroorganismen vorteilhafterweise
auch einen zusätzlichen oder zur Stressinduktion ersatzweisen Schritt der Zugabe von Oleat zum Kultivierungsmedium. Diese Zugabe führt in der Folge zu einer gesteigerten Mitochondrienproliferation in den Zellen. Da Q₁₀ in Membranen und zu grossen Teilen in der inneren Mitochondrienmembran gebildet wird, kann durch diese Zugabe von Oleat, das einen wichtigen Bestandteil der Mitochondrienmembran darstellt, über die gesteigerte Mitochondrienproliferation eine Konzentrationssteigerung von Q₁₀ in den Zellen erzielt werden.

Eine vorteilhafte Ausführungsform des erfindungsgemässen Verfahrens sieht vor, dass zur Induktion von oxidativem Stress, insbesondere am Ende der exponentiellen Wachstumsphase, pro Liter Kulturmedium 62,2 mg 13-HPOD und zwischen 1 mg/l und 5 mg/l, bevorzugt zwischen 2 mg/l und 4 mg/l, bevorzugt 3 mg/l Fe²⁺ co-inkubiert werden.

Die Co-Inkubation von Fe²⁺ und 13-HPOD bewirkt dabei eine Steigerung des oxidativen Stresses in den Mikroalgen, da durch beide Substanzen eine Lipidperoxidation ausgelöst wird, die als Initialreaktion der Membranzerstörung gilt. Auf dieses Stressereignis reagiert die Alge mit verstärkter Bildung antioxidativer Substanzen und hierbei besonders mit einer Steigerung der Co-Enzym Q₁₀-Bildung.

Photooxidativer Stress tritt in den Mikroalgen erst ab einer definierten Oberflächenbestrahlungsstärke Io am Photobioreaktor auf. Das Wachstum der im Bioreaktor kultivierten Mikroorganismen ändert jedoch die optische Dichte des Kulturmediums und somit die Lichttransmission am Bioreaktor. Um den photooxidativen Stress auch bei sich verändernden optischen Dichten des Kulturmediums konstant zu halten, erfolgt vorteilhafterweise eine Anpassung der Oberflächenbestrahlungsstärke I₀ am Bioreaktor in Abhängigkeit von der Lichttransmission. Bevorzugt erfolgt diese Anpassung der Oberflächenbestrahlungsstärke I₀ im Bereich zwischen 40 µE·m⁻²·s⁻¹ und 250 µE·m⁻²·s⁻¹. Durch das Konstanthalten der Oberflächenbestrahlungsstärke I₀ am Photobioreaktor wird die Reproduzierbarkeit der Kultivierungsbedingungen und eine gleichbleibend hohe Ausbeute an Q₁₀ aus den Mikroorganismen sichergestellt.

Wie bereits oben dargelegt, bewirkt die Zugabe von Oleat zum Kulturmedium eine gesteigerte Mitochondrienproliferation und damit eine Zunahme des aus den Mikroorganismen gewinnbaren Q₁₀. Günstigerweise erfolgt hierbei eine Zugabe von 0,05 bis 0,50 ml Oleat je Liter Kulturmedium. Eine besonders signifikante Zunahme der Q₁₀-Ausbeute zeigt sich in diesem Zusammenhang insbesondere bei Oleatmengen von 0,15 bis 0,30 ml/l, bevorzugt werden dabei dem Kulturmedium Mengen von 0,25 ml/l Oleat zugegeben.

Als empfehlenswert wird angesehen, wenn eine Supplementierung des Kulturmediums mit Oleat zwischen dem 1. und 12. Kultivierungstag erfolgt. Insbesondere erfolgt die Zugabe dabei zwischen dem 3. und 6. Kultivierungstag. Bevorzugt wird Oleat am 5. Kultivierungstag zugegeben. Die Werte können je nach kultivierter Art schwanken.

### Beispiele

Anhand der nachfolgend dargestellten Beispiele sollen die Auswirkungen der Veränderung verschiedener Kulturparameter auf die Q₁₀ -Bildung in phototrophen Mikroorganismen gezeigt werden. Als Modellorganismus wurde hierbei die in die Abteilung Rhodophyta einzuordnende Rotalge *Porphyridium purpureum* verwendet. In weiteren, nicht dargestellten Untersuchungen konnten vergleichbare Effekte bei den übrigen mit dem erfindungsgemässen Verfahren verwendbaren Mikrorganismen nachgewiesen werden.

### Beispiel 1: Co-Inkubation von P. purpureum mit 13-HPOD und Fe²⁺

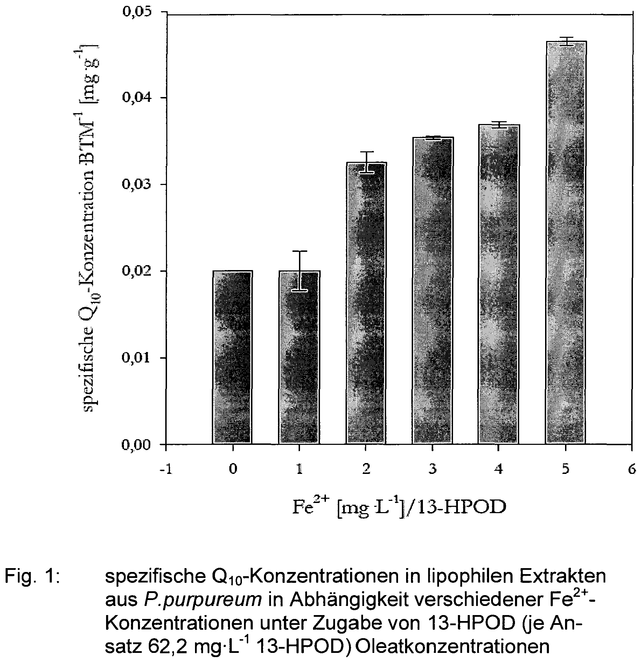

Untersucht wurde der Einfluss der Zugabe von 13-HPOD und Fe²⁺ zum Kulturmedium bei der Kultivierung von *P. purpureum.* Die Zugabe dieser beiden Substanzen bewirkt eine Erhöhung des oxidativen Stresses und hat somit unmittelbaren Einfluss auf die Q₁₀-Bildung als Stressantwort. Durch die Zugabe von Fe²⁺ in Kombination mit 13-HPOD konnte die spezifische Q₁₀-Konzentration im lipophilen Extrakt aus *P. purpureum* von 0,02 mg·g⁻¹ (Standardkultivierung) auf 0,0465 mg·g⁻¹ (5 mg·L⁻¹ Fe²⁺) gesteigert werden (vgl. Fig. 1). Dem Kultivierungsmedium wurden dabei jeweils 62,2g·L⁻¹ 13-HPOD zugegeben. Die Zugabe von 13-HPOD und verschiedenen Konzentrationen von Fe²⁺ (zwischen 0 mg·L⁻¹ und 5·mg·L⁻¹ Fe²⁺) erfolgte dabei am Ende der exponentiellen Wachstumsphase.

### Beispiel 2: Erhöhung der Oberflächenbestrahlungsstärke (OFBS)

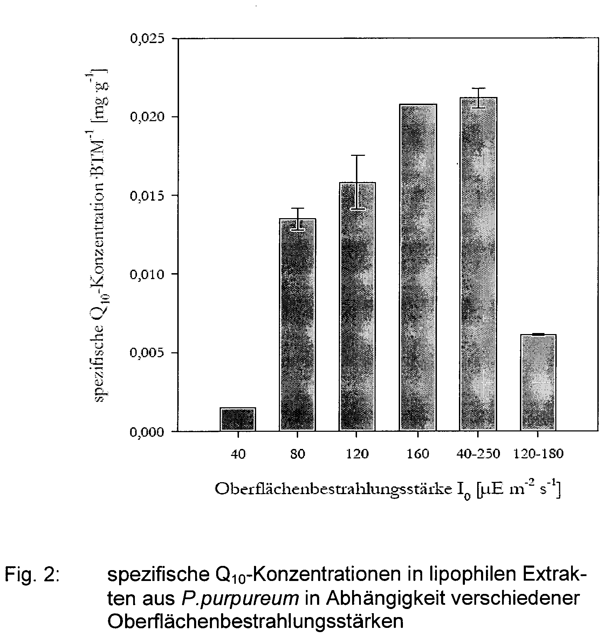

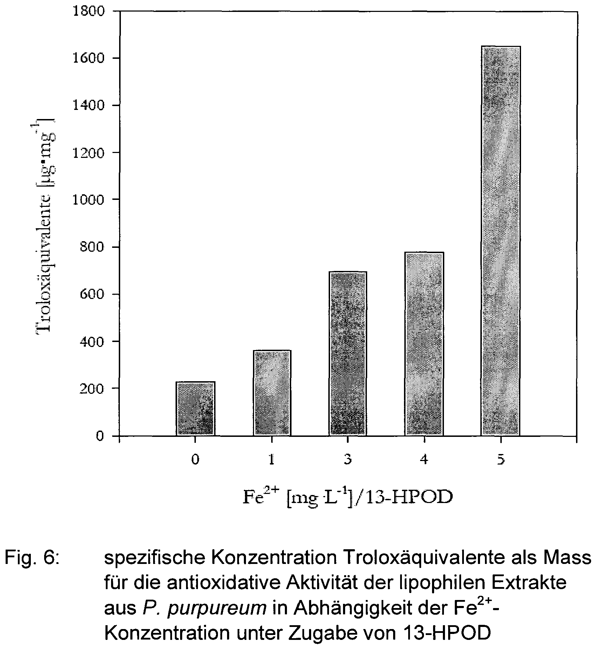

Während der Kultivierung von *P. purpureum* wurde eine Erhöhung der Oberflächenbestrahlungsstärke (OFBS) gegenüber der konstanten OBFS von 120 µE·m⁻²·s⁻¹ unter Standardkultivierungsbedingungen durchgeführt. Die Erhöhung der OBFS über 120 µE·m⁻²·s⁻¹ wirkte sich positiv auf die Q₁₀-Produktion in *P. purpureum* aus. Durch eine angepasste Erhöhung der OBFS zum Konstanthalten der Lichttransmission am Reaktor wurden hierbei die höchsten Q₁₀-Ausbeuten (0,0212 mg·g⁻¹) erzielt. Im Vergleich zur Standardkultivierung bei 120 µE·m⁻²·s⁻¹ (0,0158 mg·g⁻¹ Q₁₀) wird eine Produktionssteigerung um das 1,3-fache erzielt. Gegenüber einer Kultivierung bei 40 µE·m⁻²·s⁻¹ (1,5·10⁻³ mg·g⁻¹ Q₁₀) kann durch die angepasste Erhöhung der OFBS eine Zunahme an Q₁₀ im Organismus um den Faktor 14 erreicht werden. Eine rasche Anhebung der OFBS von 120 auf 180 µE·m⁻²·s⁻¹ bei einer optischen Dichte von 1,0 wirkt sich dagegen negativ auf die Q₁₀-Produktion aus (vgl. Fig. 2, 6. Säule).

### Beispiel 3: Zugabe von Oleat zum Kultivierungsmedium

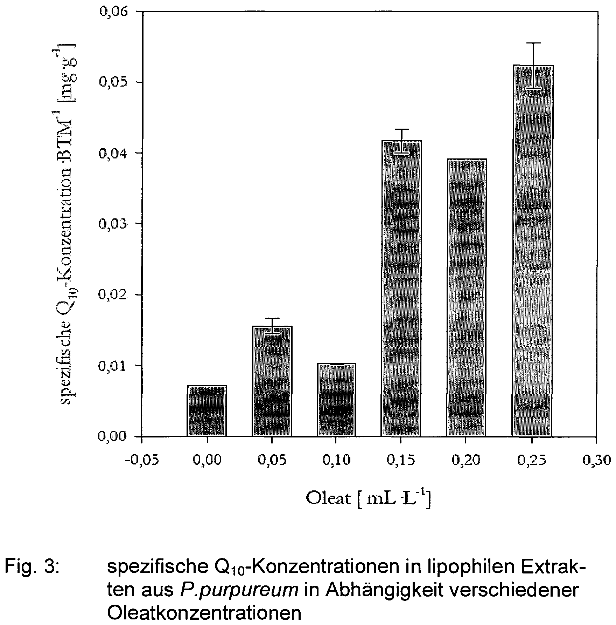

Durch die Zugabe von Oleat zum Kultivierungsmedium kann eine Steigerung der Mitochondrienproliferation in den *P. purpureum* Zellen erreicht werden. Q₁₀ kommt in Membranen und hierbei zu grossen Teilen in der inneren Mitochondrienmembran vor. Durch die Zugabe von Oleat, die einen wichtigen Bestandteil der Mitochondrienmembran darstellt, konnte eine Konzentrationssteigerung von Q₁₀ in den Zellen erzielt werden. Die Zugabe von Oleat zum Kulturmedium erfolgte am 5. Kultivierungstag. Erzielt wurde eine Produktionssteigerung von Q₁₀ in der Biomasse von *P. purpureum* um den Faktor 7 von 7,2·10⁻³ mg·g⁻¹ Q₁₀ (ohne Oleatzugabe) auf 0,05 mg·g⁻¹ Q₁₀ (0,25 ml·L⁻¹ Oleat im Medium) (vgl. Fig. 3).

### Beispiel 4: Antioxidatives Potential

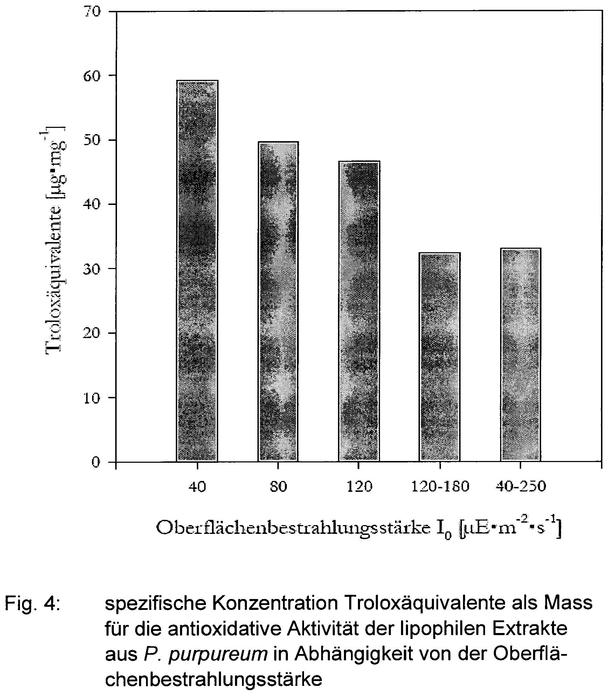

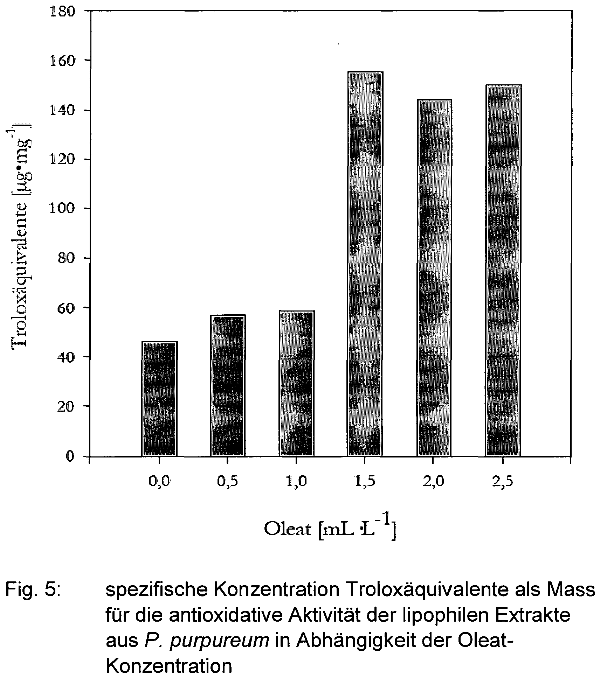

Untersucht wurde auch das antioxidative Potential der kultivierten Mikroalgen in Abhängigkeit von den jeweiligen Kulturbedingungen bzw. Medienzusätzen. Es zeigte sich hierbei, dass die Erhöhung der Oberflächenbestrahlungsstärke eine Verminderung der antioxidativen Aktivität zur Folge hat. Die antioxidative Aktivität wird hierbei in Troloxäquivalenten angegeben. Die höchste spezifische Konzentration Troloxäquivalente wird bei einer OBFS von 40 µE·m⁻²·s⁻¹ erzielt (59 µg·mg⁻¹). Im Vergleich zur Kultivierung unter Standardbedingungen (120 µE·m⁻²·s⁻¹) wird die Aktivität somit um den Faktor 1,3 erhöht (vgl. Fig. 4). Durch Supplementierung von Oleat zum Kultivierungsmedium kann vor allem im höheren Konzentrationsbereich (1,5 - 2,5 mL·L⁻¹ Oleat) ein Anstieg in der antioxidativen Kapazität der lipophilen Extrakte erzielt werden (vgl. Fig. 5). Im Vergleich zur Standardkultivierung wird das antioxidative Potential bei einer Zugabe von 1,5 ml·L⁻¹ Oleat um den Faktor 3,4 erhöht. Die Zugabe von Fe²⁺ in Kombination mit 13-HPOD bewirkt einen signifikanten Anstieg in der antioxidativen Kapazität der lipophilen Extrakte aus *P. purpureum.* Eine Fe²⁺-Konzentration von 5 mg·L⁻¹ erzielt einen Anstieg der spezifischen Konzentration Troloxäquivalente um den Faktor 7,2 im Verhältnis zu einer Kultivierung unter Standardbedingungen (vgl. Fig. 6).

### Literaturverzeichnis

Blatt, T.; Mundt, C.; Mummert, C.; Maksiuk, T; Wolber, R. Keyhani, R.; Schreiner, V.; Hoppe, U.; Schachtschabel, D. O. and Stab, F., 1999: Modulation of oxidative stresses in human aging skin. Zeitschrift für Gerontologie und Geriatrie, 32, 2, 83-88
Cenedella, R. J.; Neely, A. R. and Sexton, P., 2005: Concentration and distribution of ubiquinone (coenzyme Q), the endogenous lipid antioxidant, in the rat lens: effect of treatment with simvastatin. Molecular Vision, 11, 549-602
Chuang, Y.-C.; Chan, J. Y. H.; Chang, A. Y. W.; Sikorska, M.; Borowy-Borowski, H.; Liou, C.-W. and Chan, S. H. H., 2003: Neuroprotective effects of coenzyme Q10 at rostral ventrolateral medulla against fatality during experimental endotoxemia in the rat. Shock, 19, 5, 427-432
Ha, S.-J.; Kim, S.-Y.; Seo, J.-H.; Moon H.-J.; Lee, K.M. and Lee, J.-K., 2007: Controlling the sucrose concentration increases Coenzyme Q10 production in fed-batch culture of Agrobacterium tumefaciens. Appl. Microbiol. Biotechnol., 76, 109-116
Higdon, I and Pauling, L., 2003: Coenzyme Q10. http://lpi.oregonstate.edu/infocenter/othernuts/coq 10/index.html
Hoppe, U.; Bergemann, J.; Diembeck, W.; Ennen, J.; Gohla, S.; Harris, I.; Jacoob, J.; Kielholz, J.; Mei, W.; Pollet, D.; Schachtschabel, D.; Sauermann, G.; Schreiner, V.; Stab, F. and Steckel, F., 1999: Coenzyme Q10, a cutanous antioxidant and energizer. Biofactors, 9, 2-4, 371-378
Ishii, N.; Senoo-Matsuda, N.; Miyake, K.; Yasuda, K.; Ishii, T.; Hartman, P. S. und Furukawa, S., 2004: Coenzyme Q10 can prolong C. elegans lifespan by lowering oxidative stress. Mechanism of Ageing and Development, 125, 41-46
Jones, R. F.; Speer, H. L. and Kury, W. 1963: Studies on the Growth of the Red Alga Porphyridium cruentum. Physiologia Plantarum 16, 636-643.
König, T. 2006: Gewinnung und Charakterisierung antiviraler Wirkstoffe aus aquatischen Mikroorganismen. Dissertation.
Langsjoen, P. H.; Folkers, K.; Lyson, K.; Muratsu, K.; Lyson, T. and Langsjoen, P., 1988: Effective and safe therapy with coenzyme Q10 for cardiomyopathy. Klinische Wochenschrift, 66, 13, 583-590
Lass, A.; Agarwal, S. and Sohal, R. S., 1997: Mitochondrial Ubiquinone Homologues, Superoxide Radical Generation, and Longevity in Different Mammalian Species. The Journal of Biological Chemistry, 272, 31, 19199-19204
Lee, Y.-K., 2001: Microalgal mass culture systems and methods: Their limitation and potential. Journal of Applied Phycology, 13, 307-315
Rarbach, M., 1999: Berücksichtigung des Strahlungstransports bei der Auslegung von Photobioreaktoren. Dissertation
Shekelle, P. and Morton, S. C., 2003: Effect of Supplemental Antioxidants Vitamin C, Vitamin E, and Coenzyme Q10 for the prevention and Treatment of Cardiovascular Disease. Evidence Report/Technology Assessment, Nr. 83
Park, Y.-C.; Kim, S.-J.; Choi, J.-H.; Lee, W.-H.; Park, K.-Y.; Kawamukai, M.; Ryu, Y.-W. and Seo, J.-H. 2005: Batch and fed-batch production of coenzyme Q10 in recombinant Escherichia coli containing the decaprenyl diphosphate synthase gene from Gluconobacter suboxydans. Appl. Microbial. Biotechnol. 67, 192-196
Vormann, J., 1999: Modesubstanz oder tatsächlich Mittel zur Prävention Coenzym Q10. Kosmetische Medizin, 20, 5, 260
Walter, C.; Steinau, T.; Gerbsch, N. und Buchholz, R., 1997: Patent. "Sterilisierbarer Photobioreaktor", DE 197 47 994.4
Walter, C.; Steinau, T.; Gerbsch, N. and Buchholz, R., 2003: Monoseptic cultivation of phototrophic microorganisms - development and scale-up of a photobioreactor system with thermal sterilization. Biomolecular Engineering, 20, 4-6, 261-271
West, D., 2001: "Synthesis of Coenzyme Q10 Ubiquinone", US 20030028054

## Patentansprüche

1. Verfahren zur Steigerung des Co-Enzym Q10-Gehalts von phototrophen Mikroorganismen, die in einem Kulturmedium in einem Bioreaktor unter Standardbedingungen kultiviert werden, wobei das Wachstum der phototrophen Mikroorganismen eine exponentielle und eine stationäre Wachstumsphase aufweist,
**gekennzeichnet durch die Schritte:**
Induktion von oxidativem Stress durch
- Co-Inkubation der stressinduzierenden Substanzen 13S-Hydro-peroxy-9Z,11E-octadecadiensäure und Eisen, insbesondere Fe²⁺, mit den Mikroorganismen im Kulturmedium
und/oder
- Erhöhung der Oberflächenbestrahlungsstärke am Bioreaktor.

2. Verfahren nach Anspruch 1
**dadurch gekennzeichnet, dass**
die phototrophen Mikroorganismen Mitochondrien aufweisen und dass als weiterer Schritt eine Zugabe von Oleat zum Kulturmedium erfolgt.

3. Verfahren nach Anspruch 1,
**dadurch gekennzeichnet, dass**
die phototrophen Mikroorganismen aus den Abteilungen Rhodophyta, Chlorophyta und Haptophyta ausgewählte Mikroalgen sind, insbesondere Porphyridium purpureum, Chlorella vulgaris, Pavlova lutheri oder Cricosphaera carterae.

4. Verfahren nach einem der vorhergehenden Ansprüche,
**dadurch gekennzeichnet, dass**
zur Induktion von oxidativem Stress, insbesondere am Ende der exponentiellen Wachstumsphase, 62,2mg/l 13S-Hydroperoxy-9Z,11 E-octadecadiensäure und zwischen 1 mg/l und 5 mg/l, bevorzugt zwischen 2 mg/l und 4 mg/l, bevorzugt 3 mg/l Fe²⁺ co-inkubiert werden.

5. Verfahren nach einem der vorhergehenden Ansprüche,
**dadurch gekennzeichnet, dass**
die Erhöhung der Oberflächenbestrahlungsstärke I₀ auf Werte im Bereich zwischen 120 µE*m⁻²s*⁻¹ und 180 µE*m⁻²s*⁻¹, insbesondere auf 165 µE*m⁻²s*⁻¹ erfolgt.

6. Verfahren nach einem der vorhergehenden Ansprüche,
**dadurch gekennzeichnet, dass**
nach Erhöhung der Oberflächenbestrahlungsstärke I₀ durch Anpassung der Oberflächenbestrahlungsstärke Io ein Konstanthalten der Lichttransmission am Bioreaktor erfolgt.

7. Verfahren nach Anspruch 6,
**dadurch gekennzeichnet, dass**
die Anpassung der Oberflächenbestrahlungsstärke I₀ im Bereich zwischen 40 µE*m⁻²s*⁻¹ und 250 µE*m⁻²s*⁻¹ erfolgt.

8. Verfahren nach einem der Ansprüche 2 bis 7,
**dadurch gekennzeichnet, dass**
eine Zugabe von 0,05 bis 0,50 ml/l Oleat, insbesondere von 0,15 bis 0,30 ml/l Oleat, bevorzugt von 0,25 ml/l Oleat zum Kulturmedium erfolgt.

9. Verfahren nach einem der Ansprüche 2 bis 8,
**dadurch gekennzeichnet, dass**
die Zugabe zum Kulturmedium zwischen dem 1. und 12. Kultivierungstag, insbesondere zwischen dem 3. und 6. Kultivierungstag, bevorzugt am 5. Kultivierungstag erfolgt.

## Claims

1. A Method for increasing the coenzyme Q10-content of phototrophic microorganisms cultivated in a culture medium in a bioreactor under standard conditions, wherein growth of the phototrophic microorganisms exhibits an exponential and a steady state growth phase,
**characterized by the steps:**
- induction of oxidative stress by co-incubation of 13S-hydroperoxy-9Z,11E-octadecadienoic acid and iron, in particular Fe²⁺, with the microorganisms in the culture medium
and/or
- increasing of surface irradiation at the bioreactor.

2. The method according to claim 1,
**characterized in that**
the phototrophic microorganisms exhibit mitochondria and that addition of oleate to the culture medium is effected as a further step.

3. The method according to claim 1,
**characterized in that**
the phototrophic microorganisms are micro algae selected from the divisions Rhodophyta, Chlorophyta and Haptophyta, particularly Porphyridium purpureum, Chlorella vulgaris, Pavlova lutheri or Cricosphaera carterae.

4. The method according to anyone of the preceding claims,
**characterized in that**
for the induction of oxidative stress 62,2mg/l 13S-hydroperoxy-9Z,11E-octadecadienoic acid and between 1 mg/l and 5 mg/l, preferably between 2 mg/l and 4 mg/l, preferably 3 mg/l Fe²⁺ are co-incubated, in particular at the end of the exponential growth phase.

5. The method according to anyone of the preceding claims,
**characterized in that**
the increase of surface irradiation intensity I₀ is effected to values in the range between 120 µE*m⁻²*s⁻¹ and 180 µE*m⁻²*s⁻¹, in particular to 165 µE*m⁻²*s⁻¹.

6. The method according to anyone of the preceding claims,
**characterized in that**
after the increase of surface irradiation intensity I₀ the light transmission at the bioreactor is maintained constant by adapting the surface irradiation intensity I₀.

7. The method according to claim 6,
**characterized in that**
adapting of the surface irradiation I₀ is effected in the range between 40 µE*m⁻²*s⁻¹ and 250 µE*m⁻²*s⁻¹.

8. The method according to one of claims 2 to 7,
**characterized in that**
0,05 to 0,50 ml/l of oleate, particularly 0,15 to 0,30 ml/l of oleate, preferably 0,25 ml/l of oleate are added to the culture medium.

9. The method according to one of claims 2 to 8,
**characterized in that**
addition to the culture medium is effected between the 1^{st} and 12^{th} day of cultivation, particularly between the 3^{rd} and 6^{th} day of cultivation, preferably at the 5^{th} day of cultivation.

## Revendications

1. Procédé d'augmentation de la teneur en co-enzymes Q10 de micro-organismes phototropes cultivés dans un milieu de culture d'un bioréacteur dans des conditions normales, la croissance des micro-organismes phototropes ayant une phase exponentielle et une phase stationnaire,
procédé **caractérisé par** les étapes suivantes :
induction d'une contrainte oxydante par
- co-incubation des substances mises sous contrainte avec de l'acide 13S-Hydro-peroxy-9Z, 11E-octadécadiène et du fer, notamment Fe²⁺ avec les microorganismes du milieu de culture
et/ou
- augmentation de l'intensité d'irradiation de surface dans le bioréacteur.

2. Procédé selon la revendication 1,
**caractérisé en ce que**
les micro-organismes phototropes comportent des micro-condries et en une autre étape, on ajoute de l'oléate au milieu de culture.

3. Procédé selon la revendication 1,
**caractérisé en ce que**
les micro-organismes phototropes sont des micro-algues choisies dans la catégorie Rhodophyta, Chlorophyta et Haptophyta, notamment Porphyridium purpureum, Chlorela vulgaris, Pavlova lutheri ou Cricosphaera carterae.

4. Procédé selon l'une des revendications précédentes,
**caractérisé en ce que**
pour l'induction d'une contrainte oxydante, notamment à la fin de la phase de croissance exponentielle, on introduit 62,2mg/l d'acide 13S-Hydroperoxy-9Z,11 E-octadecadiène, et entre 1 mg/l et 5 mg/l, de préférence entre 2 mg/l et 4 mg/l et de préférence 3 mg/l de Fe²⁺.

5. Procédé selon l'une des revendications précédentes,
**caractérisé en ce qu'**
on augmente l'intensité d'irradiation de surface I₀ à des valeurs d'une plage comprise entre 120 µE*m⁻²s*⁻¹ et 180 µE*m⁻²s*⁻¹, et notamment 165 µE*m⁻²s*⁻¹.

6. Procédé selon l'une des revendications précédentes,
**caractérisé en ce qu'**
après avoir augmenté l'intensité d'irradiation de surface I₀ on maintient constante la transmission de lumière dans le bioréacteur par adaptation de l'intensité d'irradiation de surface I₀.

7. Procédé selon la revendication 6,
**caractérisé en ce qu'**
on adapte l'intensité d'irradiation de surface I₀ dans la plage comprise entre 40 µE*m⁻²s*⁻¹ et 250 µE*m⁻²s*⁻¹.

8. Procédé selon l'une des revendications 2 à 7,
**caractérisé en ce qu'**
on ajoute entre 0,05-050 ml/l d'Oléate et notamment entre 0,15-030 ml/l d'Oléate et de préférence entre 0,205 ml/l d'Oléate au milieu de culture.

9. Procédé selon l'une des revendications 2 à 8,
**caractérisé en ce que**
ajout au milieu de culture est fait entre le premier et le deuxième jours de culture, notamment entre le troisième et le sixième jour de culture et de préférence le cinquième jour de culture.
